# FASCICULE DE BREVET EUROPEEN

(11) **EP 3 128 968 B1**
(45) Date de publication et mention de la délivrance du brevet: **09.05.2018**
(21) Numéro de dépôt: 15714848.7
(22) Date de dépôt: 03.04.2015
(51) Int. Cl.: A61F 13/06, A61F 13/14, A61F 13/08, A61F 13/10

(54) **VÊTEMENT POUR LE TRAITEMENT D'AFFECTIONS TISSULAIRES CUTANÉES ET SOUS-CUTANÉES ET DE RÉCUPÉRATION SPORTIVE, COMPRENANT UN CORPS TUBULAIRE**
KLEIDUNGSSTÜCK ZUR BEHANDLUNG VON HAUT- UND UNTERHAUTGEWEBEERKRANKUNGEN UND ZUR SPORTREHABILITATION MIT EINEM SCHLAUCHFÖRMIGEN KÖRPER
ITEM OF CLOTHING FOR THE TREATMENT OF SKIN AND SUBCUTANEOUS TISSUE DISORDERS AND SPORTS RECOVERY, COMPRISING A TUBULAR BODY

(30) Priorité: 09.04.2014 FR 1453171
(43) Date de publication de la demande: 15.02.2017
(73) Titulaire: Thuasne, 92300 Levallois Perret (FR)
(72) Inventeur: GALLIEN, Nathalie, 42100 Saint-Etienne (FR); DE MONCUIT, Henri, Bakersfield, California 93309 (US); CONVERT, Reynald, 69006 Lyon (FR)
(74) Mandataire: Lavoix
(86) Numéro de dépôt international: PCT/EP2015/097006
(87) Numéro de publication internationale: WO 2015/155372

(56) Documents cités:
- EP-B1- 1 926 458
- US-A- 5 743 866

## Description

La présente invention concerne un vêtement pour le traitement d'affections tissulaires cutanées et sous-cutanées et de récupération sportive, du type comprenant un corps tubulaire avec au moins une partie relativement inélastique, la ou chaque partie inélastique présentant une couche intérieure définissant une face intérieure de la partie inélastique, ladite couche intérieure comportant une pluralité d'élément saillants espacés les uns des autres.

Un tel vêtement est connu par exemple de EP 1 926 458. Un autre vêtement pour le traitement d'affections tissulaires cutanées et sous-cutanées est également connu de US 5 743 866.

Cependant, les vêtements actuels ne donnent pas entière satisfaction. En effet, le corps tubulaire est le plus souvent intégralement formé par la partie inélastique, ce dont il résulte que le vêtement ne peut pas être ajusté à la partie du corps du patient à laquelle est destiné le traitement. Il existe alors un risque que la pression exercée sur la partie du corps du patient soit excessive ou trop faible, avec pour conséquence un mauvais traitement du patient. En outre, de tels vêtements ne sont pas faciles à enfiler car ils ne présentent pas une extensibilité suffisante pour permettre de glisser un membre facilement à l'intérieur du corps tubulaire.

Un objectif de l'invention est de permettre l'ajustement du vêtement. D'autres objectifs sont de permettre l'ajustement de la pression exercée par le vêtement sur le corps du patient, de faciliter son enfilage, et d'assurer un traitement homogène de la partie du corps à soigner.

A cet effet, l'invention a pour objet, selon un premier aspect, un corps tubulaire comprenant également une partie relativement élastique s'étendant d'une première extrémité axiale à une extrémité axiale opposée du corps tubulaire et reliant un premier bord de la partie inélastique à un deuxième bord de la partie inélastique, et en ce que le vêtement comprend en outre :
- un rabat relativement inélastique présentant une couche intérieure définissant une face intérieure du rabat, ladite couche intérieure comportant une pluralité d'élément saillants espacés les uns des autres, le rabat étant solidaire d'au moins une partie du premier bord de la partie inélastique, et
- un dispositif d'accrochage réversible du rabat à au moins une partie du deuxième bord de la partie inélastique, adapté pour que le rabat chevauche la partie élastique lorsqu'il est accroché.

L'invention a également pour objet, selon un deuxième aspect, un vêtement du type précité, dans lequel le corps tubulaire comprend également une pluralité de parties relativement élastiques, s'étendant chacune d'une première extrémité axiale à une extrémité axiale opposée du corps tubulaire et reliant chacune un premier bord d'une partie inélastique à un deuxième bord d'une autre partie inélastique, et en ce que le vêtement comprend en outre, pour chaque partie élastique :
- un rabat relativement inélastique présentant une couche intérieure définissant une face intérieure du rabat, ladite couche intérieure comportant une pluralité d'élément saillants espacés les uns des autres, le rabat étant solidaire d'au moins une partie du premier bord de partie inélastique, et
- un dispositif d'accrochage réversible du rabat à au moins une partie du deuxième bord de partie inélastique, adapté pour que le rabat chevauche la partie élastique lorsqu'il est accroché.

De préférence, les parties élastiques et inélastiques sont disposées en alternance les unes avec les autres

Suivant des modes de réalisation particuliers de l'invention, le vêtement selon le premier ou le deuxième aspect présente également l'une ou plusieurs des caractéristiques suivantes, prise(s) isolément ou suivant toute(s) combinaison(s) techniquement possible(s) :
- la ou chaque partie inélastique et le ou chaque rabat comprennent chacun une couche extérieure en matériau relativement inélastique à laquelle la couche intérieure est fixée ;
- la couche intérieure est fixée à la couche extérieure par collage ;
- la couche extérieure est en matière tissée ou tricotée ;
- le dispositif d'accrochage réversible comprend au moins un organe de fixation primaire solidaire du rabat et au moins un organe de fixation secondaire solidaire du deuxième bord de partie inélastique, le ou chaque organe de fixation primaire étant adapté pour coopérer avec un organe de fixation secondaire respectif de façon à permettre l'accrochage du rabat au deuxième bord de partie inélastique dans différentes positions ;
- un dispositif de repérage de la position du rabat relativement au deuxième bord de partie inélastique ;
- le dispositif de repérage comprend un premier repère porté par le rabat, respectivement par la partie inélastique, et une pluralité de deuxièmes repères portés par la partie inélastique, respectivement par le rabat, chaque deuxième repère étant associé à une position propre d'accrochage du rabat au deuxième bord, le premier repère étant adapté pour, dans chaque position d'accrochage du rabat au deuxième bord, être disposé en correspondance avec le deuxième repère associé à ladite position ;
   - le dispositif d'accrochage est un dispositif autoagrippant ;
   - les éléments saillant comprennent des morceaux de mousse, des carrés de tricot tridimensionnel, des coussinets remplis de liquide, ou des pavés pleins en polymère.

D'autres caractéristiques et avantages de l'invention apparaîtront à la lecture de la description qui va suivre, donnée uniquement à titre d'exemple et faite en se référant aux dessins annexés, dans lesquels :
- la Figure 1 est une vue schématique en élévation d'un vêtement selon un premier exemple de réalisation de l'invention,
- la Figure 2 est une vue en coupe du vêtement de la Figure 1, selon un plan de coupe marqué II-II sur la Figure 1, le vêtement étant desserré,
- la Figure 3 est une vue similaire à celle de la Figure 2, le vêtement étant serré,
- la Figure 4 est une vue schématique en élévation d'un vêtement selon un deuxième exemple de réalisation de l'invention, et
- la Figure 5 est une vue schématique en coupe d'un vêtement selon un troisième exemple de réalisation de l'invention.

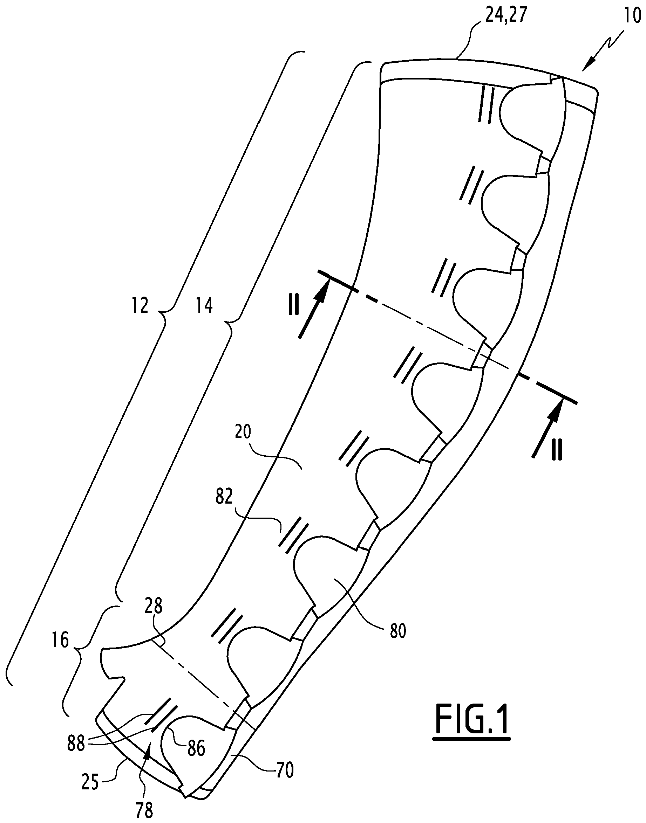

Le vêtement 10 représenté sur la Figure 1 est destiné au traitement d'affections tissulaires cutanées et sous-cutanées et en particulier pour le traitement de divers types d'oedèmes ou d'infiltrations tissulaires excessives. Le vêtement 10 peut également être utilisé pour la récupération sportive après effort.

Dans l'exemple représenté, le vêtement 10 est un manchon destiné à être enfilé sur le bras d'un patient. En variante, le vêtement 10 est un bas-cuisse, une chaussette, un gant, une mitaine, un panty, un gilet, un manchon tour de cou ou un slip.

Le vêtement 10 comprend un corps tubulaire 12. Ce corps tubulaire 12 comprend un tronçon fermé 14 et un tronçon ouvert 16.

Les tronçons fermé 14 et ouvert 16 forment chacun un corps tubulaire. Ils sont chacun ouverts à leurs extrémités axiales respectives. Toute section transversale du tronçon fermé 14 est une section fermée. Le tronçon ouvert 16 présente une fente (non représentée) reliant les extrémités axiales dudit tronçon 16 l'une à l'autre.

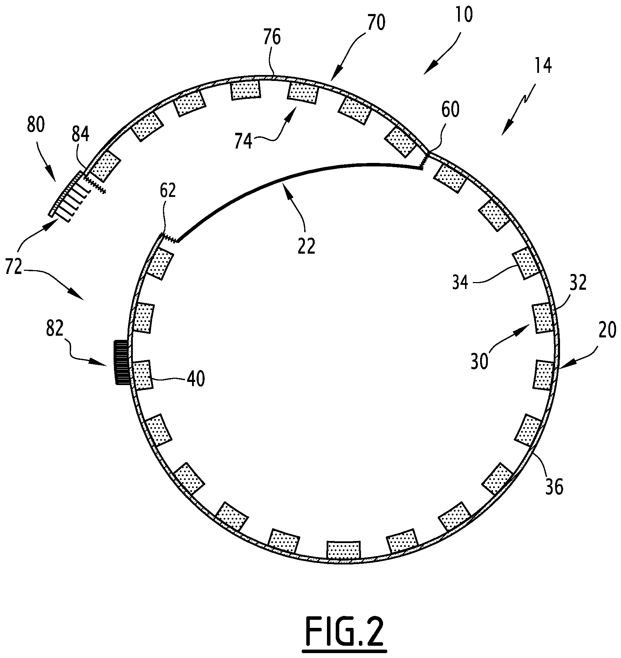

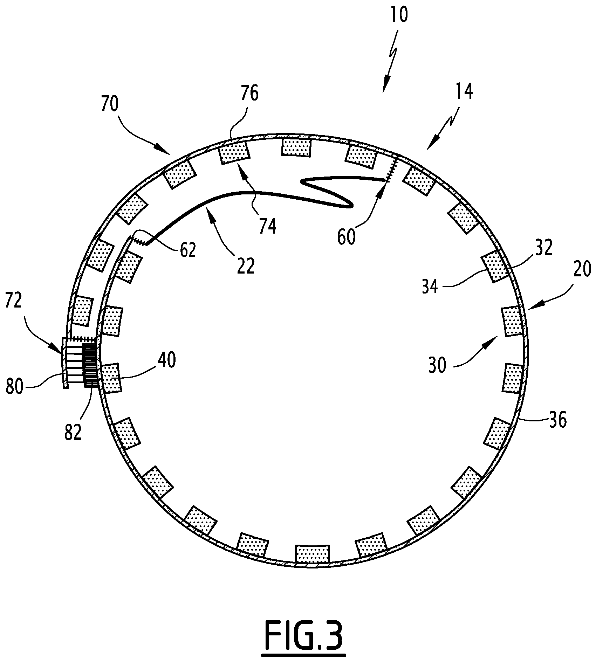

En référence aux Figures 2 et 3, le corps tubulaire 12 est formé d'une partie inélastique 20 et d'une partie élastique 22. Ici et dans la suite, les termes « élastique » et « inélastique » sont à entendre comme des termes relatifs : la partie inélastique 20 est relativement inélastique lorsqu'elle est comparée à la partie élastique 22, mais cela n'exclut pas une certaine élasticité propre de la partie inélastique 20. De préférence, la différence d'allongement entre les parties élastique 22 et inélastique 20, mesurée selon la norme EN 14704-1, est supérieure à 50% sous une force de 5N/cm.

En référence à la Figure 1, la partie inélastique 20 s'étend d'une extrémité axiale 24 du corps 12 à l'extrémité axiale 25 opposée. La partie inélastique 20 s'étend donc notamment d'une extrémité axiale 27 du tronçon fermé 14 à l'extrémité axiale 28 opposée.

La partie élastique 22 s'étend exclusivement dans le tronçon fermé 14. Elle s'étend en particulier d'une extrémité axiale 27 du tronçon fermé 14 à l'extrémité axiale 28 opposée. Le tronçon ouvert 16 est ainsi constitué par la seule partie inélastique 20.

De retour à la Figure 2, la partie inélastique 20 comprend une couche intérieure 30 et une couche extérieure 32 fixées l'une à l'autre. La couche intérieure 30 est plus proche de l'axe central du corps tubulaire 12 que la couche extérieure 32. En particulier, la couche intérieure 30 définit une face intérieure 34 de la partie inélastique 20, orientée vers l'axe central du corps 12, et la couche extérieure 32 définit une face extérieure 36 de la partie inélastique 20, orientée à l'opposé de l'axe central du corps 12.

La couche extérieure 32 est réalisée en matière textile, tissée, tricotée ou non-tissée, complexée ou non complexée, avec un allongement de préférence inférieur à 60% mesuré selon la norme EN 14704-1 sous une force de 5N/cm.

La couche intérieure 30 est fixée à la couche extérieure 32, par exemple par collage, couture, soudure ou autre. Elle comprend essentiellement une pluralité d'éléments saillants 40.

Dans l'exemple représenté, les éléments saillants 40 sont des pavés de mousse. En variante, les éléments saillants 40 sont des carrés de tricot tridimensionnel, des coussinets remplis d'un liquide, ou des pavés pleins en polymère, par exemple en silicone ou en élastomère thermoplastique (mieux connu sous l'acronyme TPE).

Dans l'exemple représenté, les éléments saillants 40 sont directement posés sur la couche extérieure 32 et définissent la face intérieure 34 de la partie inélastique 20. En variante (non représentée), la couche intérieure 30 comprend une nappe intérieure, de préférence en matière textile, recouvrant les éléments saillants 40 et définissant la face intérieure 34 ; ainsi, la peau du patient est protégée d'un contact direct avec les éléments saillants 40. En variante encore (non représentée), la couche intérieure 30 comprend, en plus de la nappe intérieure, une nappe extérieure, de préférence également en matière textile, interposée entre les éléments saillants 40 et la couche extérieure 32, les deux nappes étant solidaires entre elles.

De retour aux Figures 2 et 3, la partie élastique 22 relie un premier bord 60 de la partie inélastique 20 à un deuxième bord 62 opposé de ladite partie inélastique 20. Elle est de préférence cousue audits bords 60, 62.

La partie élastique 22 a de préférence une valeur d'allongement comprise entre 100 et 200% selon la norme EN 14704-1 lorsque soumise à une force de 5N/cm. Elle est par exemple en tricot Jersey Polyamide Élasthanne avec une masse surfacique de 180g/m².

Grâce à cette partie élastique 22, l'enfilage du vêtement 10 est facilité en permettant d'agrandir le diamètre du corps tubulaire 12 pour glisser un membre à l'intérieur.

Le vêtement 10 comprend en outre un rabat 70 solidaire du premier bord 60 de la partie inélastique 20, un dispositif 72 d'accrochage réversible du rabat 70 au deuxième bord 62 de la partie inélastique 20, et un dispositif 78 (Figure 1) de repérage de la position du rabat 70 relativement au deuxième bord 62 de la partie inélastique 20.

Le rabat 70 est relativement inélastique. Il comprend une couche intérieure 74 et une couche extérieure 76 fixées l'une à l'autre, la couche intérieure 74 étant identique à la couche intérieure 30 de la partie inélastique 20 et la couche extérieure 76 étant identique à la couche extérieure 32 de la partie inélastique 20. De préférence, la couche intérieure 74 est un prolongement de la couche intérieure 30 de la partie inélastique 20 au-delà du premier bord 60 et la couche extérieure 76 est un prolongement de la couche extérieure 32 de la partie inélastique 20 au-delà du premier bord 60 ; le rabat 70 constitue ainsi une prolongation de la partie inélastique 20 au-delà de son premier bord 60.

Le dispositif d'accrochage 72 est adapté pour que, lorsqu'il est accroché, le rabat 70 chevauche la partie élastique 22 dans le tronçon fermé 14 et la fente dans le tronçon ouvert 16. Ainsi, lorsque le rabat 70 est accroché, la partie du corps à traiter est intégralement entourée par les couches intérieures 30, 74 des partie inélastique 20 et rabat 70, ce qui assure un traitement homogène de cette partie du corps.

De préférence, le dispositif d'accrochage 72 est également adapté pour que, lorsqu'il est accroché, le rabat 70 chevauche partiellement la partie inélastique 20.

Le dispositif d'accrochage 72 comprend une pluralité d'organes de fixation primaires 80, solidaires du rabat 70, et une pluralité d'organes de fixation secondaires 82, solidaires du deuxième bord 62 de la partie inélastique 20, chaque organe de fixation primaire 80 étant adapté pour coopérer avec un organe de fixation secondaire 82 respectif pour permettre l'accrochage du rabat 70 au deuxième bord 62 dans différentes positions. Les organes de fixation primaires et secondaires 80, 82 sont en particulier adaptés pour accrocher le rabat 70 au deuxième bord 62 dans une première position, dans laquelle le bord libre 84 du rabat 70, opposé au bord par lequel le rabat 70 est lié à la partie inélastique 20, affleure le deuxième bord 62, et dans une deuxième position, dans laquelle le bord libre 84 du rabat 70 chevauche le deuxième bord 62, comme représenté sur la Figure 3.

Il est ainsi possible d'ajuster le diamètre du corps tubulaire 12 lors de l'accrochage du rabat 70, ce qui permet d'adapter le vêtement 10 à la corpulence du patient et de régler la force de compression appliquée sur le corps par le vêtement 10.

Chaque organe de fixation primaire 80 est fixé, par exemple cousu, collé ou soudé, au bord libre 84 du rabat 70, et fait saillie depuis ce bord libre 84 à l'opposé du premier bord 60.

Chaque organe de fixation secondaire 82 est, dans l'exemple représenté, une pièce rapportée sur la face extérieure 36 de la partie inélastique 20 ; il est alors cousu, collé ou soudé à la couche extérieure 32. En variante, l'organe de fixation secondaire 82 forme au moins une partie de la couche extérieure 32.

Dans l'exemple représenté, le dispositif de fixation 72 est formé par un dispositif autoagrippant, en particulier de type boucles et crochets (mieux connu sous la marque Velcro ®). L'organe de fixation primaire 80 comporte alors de préférence les crochets et l'organe de fixation 82 comporte les boucles.

En variante (non représentée), le dispositif de fixation 72 est formé par un système boucle et lanière, une pièce plastique, des crochets, un système de bouton-pression, ou un système de préhension par aimant.

De retour à la Figure 1, le dispositif de repérage 78 comprend un premier repère 86 porté par le rabat 70 et une pluralité de deuxièmes repères 88 portés par la face extérieure 36 de la partie inélastique 20, chaque deuxième repère 88 étant associé à une position respective d'accrochage du rabat 70 au deuxième bord 62. Le premier repère 86 est adapté pour, dans chaque position d'accrochage du rabat 70 au deuxième bord 62, être disposé en correspondance avec le deuxième repère 88 associé à ladite position.

Dans l'exemple représenté, le premier repère 86 est formé par l'extrémité libre de l'organe de fixation primaire 80 et chaque deuxième repère 88 est formé par une marque, en particulier un trait parallèle au bord 62, marquée sur l'organe de fixation secondaire 82. Le premier repère 86 est alors mis en correspondance avec un deuxième repère 88 lorsqu'il affleure la marque constituant ce deuxième repère 88. En variante, chaque deuxième repère 88 est réalisé par impression, sérigraphie, couture, emporte-pièce, ou toute autre méthode de marquage directement sur la couche extérieure 32 de la partie inélastique 20.

Il est ainsi possible pour l'utilisateur de se rendre compte aisément de la force de serrage du vêtement 10.

Un procédé d'utilisation du vêtement 10 va maintenant être décrit, en référence aux Figures 1 à 3.

L'utilisateur enfile tout d'abord le vêtement 10, le rabat 70 n'étant alors pas accroché au deuxième bord 62 de la partie inélastique 20. Lorsque le corps tubulaire 12 passe autour de portions du corps plus larges que la partie destinée à être traitée, le corps tubulaire 12 s'élargit, grâce à l'élasticité de la partie élastique 22. Le vêtement 10 s'enfile ainsi facilement.

Une fois le vêtement 10 en place, l'utilisateur rabat le rabat 70 par-dessus la partie élastique 22. Puis il positionne l'extrémité libre 84 du rabat 70 par rapport au deuxième bord 62 de la partie inélastique 20 de façon à mettre en correspondance le premier repère 86 du dispositif de repérage 78 avec un deuxième repère 88 prédéterminé, en fonction de la force de compression qu'il souhaite appliquer à la partie du corps à traiter. Une fois cette mise en correspondance effectuée, l'utilisateur accroche le rabat 70 au deuxième bord 62. L'utilisateur peut ainsi facilement régler la force de compression qu'il souhaite appliquer sur la partie du corps à traiter.

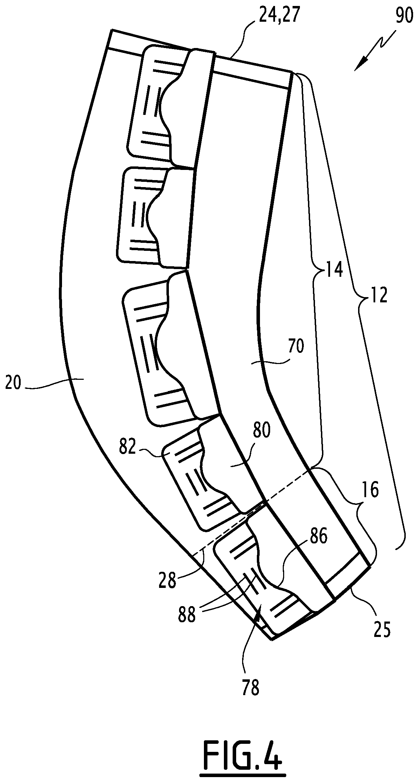

Le vêtement 90 de la Figure 4 ne se distingue du vêtement 10 de la Figure 1 que par la forme des organes de fixation primaires 80. Les mêmes signes de référence ont été repris pour les éléments identiques à ceux du vêtement 10.

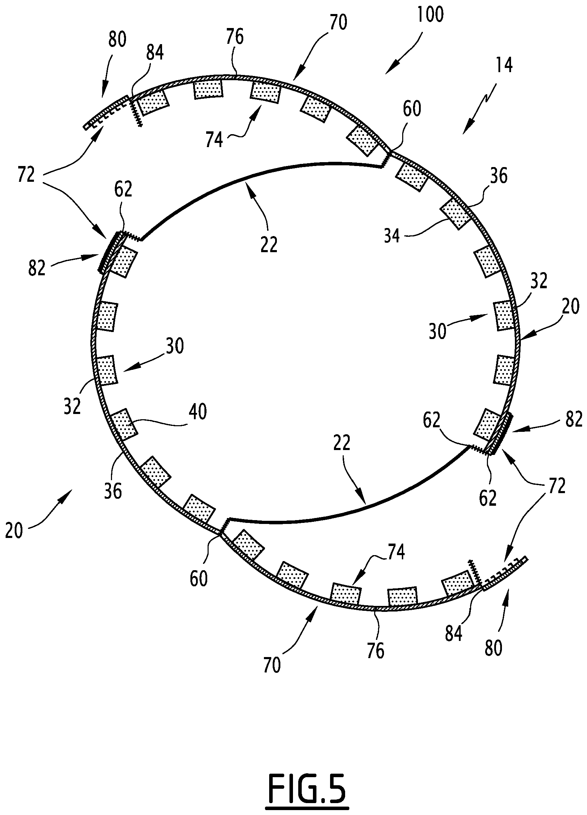

Le vêtement 100 de la Figure 5 étant pour l'essentiel identique au vêtement 10 de la Figure 1, les mêmes signes de référence ont été repris pour les éléments identiques. Il s'en distingue uniquement en ce que le corps tubulaire 12 comprend une pluralité de parties inélastiques 20 espacées les unes des autres et une pluralité de parties élastiques 22 reliant les parties inélastiques 20 les unes aux autres, et en ce qu'il comprend une pluralité de rabats 70, chacun adapté pour chevaucher une partie élastique 22 respective lorsqu'il est accroché. En particulier, chaque partie élastique 22 relie un premier bord 60 d'une partie inélastique 20 à un deuxième bord 62 d'une autre partie inélastique 20, chaque rabat 70 est solidaire d'un premier bord 60 d'une partie inélastique 20 et est adapté pour être accroché à un deuxième bord 62 d'une autre partie inélastique 20, et les parties élastiques 20 et inélastiques 22 sont disposées en alternance les unes avec les autres.

Ce mode de réalisation est avantageux lorsque le corps tubulaire 12 a un grand diamètre.

Le corps tubulaire 12 a été décrit comme comprenant un tronçon fermé et un tronçon ouvert. Il est cependant entendu que l'invention s'applique également à tout vêtement comprenant uniquement un tronçon fermé, ainsi qu'à un vêtement comprenant une pluralité de tronçons fermés dont les parties élastiques respectives ne sont pas alignées les unes avec les autres.

## Revendications

1. Vêtement (10, 90) pour le traitement d'affections tissulaires cutanées et sous-cutanées et de récupération sportive, comprenant un corps tubulaire (14) avec une partie relativement inélastique (20), ladite partie inélastique (20) présentant une couche intérieure (30) définissant une face intérieure (34) de la partie inélastique (20), ladite couche intérieure (30) comportant une pluralité d'élément saillants (40) espacés les uns des autres,
**caractérisé en ce que** le corps tubulaire (14) comprend également une partie relativement élastique (22) s'étendant d'une première extrémité axiale (27) à une extrémité axiale opposée (28) du corps tubulaire (14) et reliant un premier bord (60) de la partie inélastique (20) à un deuxième bord (62) de la partie inélastique (20), et **en ce que** le vêtement (10) comprend en outre :
- un rabat (70) relativement inélastique présentant une couche intérieure (74) définissant une face intérieure du rabat (70), ladite couche intérieure (74) comportant une pluralité d'élément saillants (40) espacés les uns des autres, le rabat (70) étant solidaire d'au moins une partie du premier bord (60) de la partie inélastique (20), et
- un dispositif (72) d'accrochage réversible du rabat (70) à au moins une partie du deuxième bord (62) de la partie inélastique (20), adapté pour que le rabat (70) chevauche la partie élastique (22) lorsqu'il est accroché.

2. Vêtement (100) pour le traitement d'affections tissulaires cutanées et sous-cutanées et de récupération sportive, comprenant un corps tubulaire (14) avec une pluralité de parties relativement inélastiques (20) espacées les unes des autres et présentant chacune une couche intérieure (30) définissant une face intérieure (34) de ladite partie inélastique (20), ladite couche intérieure (30) comportant une pluralité d'élément saillants (40) espacés les uns des autres,
**caractérisé en ce que** le corps tubulaire (14) comprend également une pluralité de parties relativement élastiques (22), s'étendant chacune d'une première extrémité axiale (27) à une extrémité axiale opposée (28) du corps tubulaire (14) et reliant chacune un premier bord (60) d'une partie inélastique (20) à un deuxième bord (62) d'une autre partie inélastique (20), et **en ce que** le vêtement (10) comprend en outre, pour chaque partie élastique (22) :
- un rabat (70) relativement inélastique présentant une couche intérieure (74) définissant une face intérieure du rabat (70), ladite couche intérieure (74) comportant une pluralité d'élément saillants (40) espacés les uns des autres, le rabat (70) étant solidaire d'au moins une partie du premier bord de partie inélastique (60), et
- un dispositif (72) d'accrochage réversible du rabat (70) à au moins une partie du deuxième bord de partie inélastique (62), adapté pour que le rabat (70) chevauche la partie élastique (22) lorsqu'il est accroché.

3. Vêtement (100) selon la revendication 2, dans lequel les parties élastiques et inélastiques (20, 22) sont disposées en alternance les unes avec les autres.

4. Vêtement (10, 90, 100) selon l'une quelconque des revendications précédentes, dans lequel la ou chaque partie inélastique (20) et le ou chaque rabat (70) comprennent chacun une couche extérieure (32, 76) en matériau relativement inélastique à laquelle la couche intérieure (30, 74) est fixée.

5. Vêtement (10, 90, 100) selon la revendication 4, dans lequel la couche intérieure (30, 74) est fixée à la couche extérieure (32, 76) par collage

6. Vêtement (10, 90, 100) selon la revendication 4 ou 5, dans lequel la couche extérieure (32, 76) est en matière tissée ou tricotée.

7. Vêtement (10, 90, 100) selon l'une quelconque des revendications précédentes, dans lequel le dispositif d'accrochage réversible (72) comprend au moins un organe de fixation primaire (80) solidaire du rabat (70) et au moins un organe de fixation secondaire (82) solidaire du deuxième bord de partie inélastique (62), le ou chaque organe de fixation primaire (80) étant adapté pour coopérer avec un organe de fixation secondaire (82) respectif de façon à permettre l'accrochage du rabat (70) au deuxième bord de partie inélastique (62) dans différentes positions.

8. Vêtement (10, 90, 100) selon la revendication 7, comprenant un dispositif (78) de repérage de la position du rabat (70) relativement au deuxième bord de partie inélastique (62).

9. Vêtement (10, 90, 100) selon la revendication 8, dans lequel le dispositif de repérage (78) comprend un premier repère (86) porté par le rabat (70), respectivement par la partie inélastique, et une pluralité de deuxièmes repères (88) portés par la partie inélastique (20), respectivement par le rabat, chaque deuxième repère (88) étant associé à une position propre d'accrochage du rabat (70) au deuxième bord (62), le premier repère (86) étant adapté pour, dans chaque position d'accrochage du rabat (70) au deuxième bord (62), être disposé en correspondance avec le deuxième repère (88) associé à ladite position.

10. Vêtement (10, 90, 100) selon l'une quelconque des revendications précédentes, dans lequel le dispositif d'accrochage (72) est un dispositif autoagrippant.

11. Vêtement (10, 90, 100) selon l'une quelconque des revendications précédentes, dans lequel les éléments saillant (40) comprennent des morceaux de mousse, des carrés de tricot tridimensionnel, des coussinets remplis de liquide, ou des pavés pleins en polymère.

## Patentansprüche

1. Bekleidungsstück (10, 90) zur Behandlung von kutanen und subkutanen Gewebeleiden und zur Sport-Rehabilitation, aufweisend einen rohrförmigen Körper (14) mit einem relativ unelastischen Abschnitt (20), wobei der besagte unelastische Abschnitt (20) eine innere Schicht (30) hat, die eine Innenseite (34) der unelastischen Schicht (20) definiert, wobei die besagte innere Schicht (30) eine Mehrzahl von Vorsprungelementen (40) aufweist, die im Abstand voneinander sind,
**dadurch gekennzeichnet, dass** der rohrförmige Körper (14) auch aufweist einen relativ elastischen Abschnitt (22), der sich von einem ersten Axialende (27) bis zu einem entgegengesetzten Axialende (28) des rohförmigen Körpers (14) erstreckt und der einen ersten Rand (60) des unelastischen Abschnitts (20) mit einem zweiten Rand (62) des unelastischen Abschnitts (20) verbindet, und dass das Kleidungsstück (10) ferner aufweist:
- eine relativ unelastische Lasche (70), die eine innere Schicht (74) hat, die eine Innenseite der Lasche (70) definiert, wobei die besagte innere Schicht (74) eine Mehrzahl von Vorsprungelementen (40) aufweist, die im Abstand voneinander sind, wobei die Lasche (70) wenigstens mit einem Abschnitt des ersten Rands (60) des unelastischen Abschnitts (20) fest verbunden ist, und
- eine Vorrichtung (72) zur reversiblen Ankopplung der Lasche (70) mit wenigstens einem Abschnitt des zweiten Rands (62) des unelastischen Abschnitts (20), die angepasst ist damit die Lasche (70) auf dem elastischen Abschnitt (22) aufliegt, wenn sie angekoppelt ist.

2. Kleidungsstück (100) zur Behandlung von kutanen und subkutanen Gewebeleiden und zur Sport-Rehabilitation, aufweisend einen rohrförmigen Körper (14) mit einer Mehrzahl von relativ unelastischen Abschnitten (20), die im Abstand voneinander sind und jeweils eine innere Schicht (30) haben, die eine Innenseite (34) des besagten unelastischen Abschnitts (20) definieren, wobei die besagte innere Schicht (30) eine Mehrzahl von Vorsprungelementen (40) aufweist, die im Abstand voneinander sind,
**dadurch gekennzeichnet, dass** der rohrförmige Körper (14) auch eine Mehrzahl von relativ elastischen Abschnitten (22) aufweist, die sich jeweils von einem ersten Axialabschnitt (27) zu einem entgegengesetzten Axialabschnitt (28) des rohrförmigen Körpers (14) erstrecken und jeweils einen ersten Rand (60) eines unelastischen Abschnitts (20) mit einem zweiten Rand (62) eines anderen unelastischen Abschnitts (20) verbinden, und dass das Kleidungsstück (10) ferner aufweist, für jeden elastischen Abschnitt (22):
- eine relativ unelastische Lasche (70), die eine innere Schicht (74) hat, die eine Innenseite der Lasche (70) definiert, wobei die besagte innere Schicht (74) eine Mehrzahl von Vorsprungelementen (40) aufweist, die im Abstand voneinander sind, wobei die Lasche (70) wenigstens mit einem Abschnitt des ersten Rands des unelastischen Abschnitts (60) fest verbunden ist, und
- eine Vorrichtung (72) zur reversiblen Kopplung der Lasche (70) mit wenigstens einem Abschnitt des zweiten Rands des unelastischen Abschnitts (62), die angepasst ist damit die Lasche (70) auf dem elastischen Abschnitt (22) aufliegt, wenn sie gekoppelt ist.

3. Kleidungsstück (100) gemäß Anspruch 2, wobei die elastischen und unelastischen Abschnitte (20, 22) abwechselnd miteinander angeordnet sind.

4. Kleidungsstück (10, 90, 100) gemäß irgendeinem der vorhergehenden Ansprüche, wobei der oder jeder unelastische Abschnitt (20) und die oder jede Lasche (70) jeweils aufweisen eine äußere Schicht (32, 76) aus einem relativ unelastischen Material, an welcher die innere Schicht (30, 74) fixiert ist.

5. Kleidungsstück (10, 90, 100) gemäß Anspruch 4, wobei die innere Schicht (30, 74) an der äußeren Schicht (32, 76) durch Klebung fixiert ist.

6. Kleidungsstück (10, 90, 100) gemäß Anspruch 4 oder 5, wobei die äußere Schicht (32, 76) aus einem gewebten oder gestrickten Material ist.

7. Kleidungsstück (10, 90, 100) gemäß irgendeinem der vorhergehenden Ansprüche, wobei die Vorrichtung zur reversiblen Kopplung (72) aufweist wenigstens ein Primär-Fixierorgan (80), das mit der Lasche (70) fest verbunden ist, und wenigstens ein Sekundär-Fixierorgan (82), das mit dem zweiten Rand des unelastischen Abschnitts (62) fest verbunden ist, wobei das oder jedes Primär-Fixierorgan (80) angepasst ist zum Zusammenwirken mit einem zugeordneten Sekundär-Fixierorgan (82) in einer Weise, um die Kopplung der Lasche (70) mit dem zweiten Rand des unelastischen Abschnitts (62) in unterschiedlichen Positionen zu ermöglichen.

8. Kleidungsstück (10, 90, 100) gemäß Anspruch 7, aufweisend eine Vorrichtung (78) zur Markierung der Position der Lasche (70) relativ zu dem zweiten Rand des unelastischen Abschnitts (62).

9. Kleidungsstück (10, 90, 100) gemäß Anspruch 8, wobei die Markierungs-Vorrichtung (78) aufweist eine erste Markierung (86), die von der Lasche (70), respektive von dem unelastischen Abschnitt, getragen wird, und eine Mehrzahl von zweiten Markierungen (88), die von dem unelastischen Abschnitt (20), respektive von der Lasche, getragen werden, wobei jede zweite Markierung (88) einer sauberen Position zur Kopplung der Lasche (70) mit dem zweiten Rand (62) zugeordnet ist, wobei die erste Markierung (86) angepasst ist, um, in jeder Position zur Kopplung der Lasche (70) mit dem zweiten Rand (62), korrespondierend mit der zweiten Markierung (88) angeordnet zu sein, die der besagten Position zugeordnet ist.

10. Kleidungsstück (10, 90, 100) gemäß irgendeinem der vorhergehenden Ansprüche, wobei die Kopplungs-Vorrichtung (72) eine Klett-Vorrichtung ist.

11. Kleidungsstück (10, 90, 100) gemäß irgendeinem der vorhergehenden Ansprüche, wobei die Vorsprungelemente (40) aufweisen Schaumstücke, Karrees aus dreidimensionalem Strickstoff, Flüssigkeit-gefüllte Kissen oder Vollpolymerblöcke.

## Claims

1. An item of clothing (10, 90) for the treatment of skin and subcutaneous tissue disorders and sports recovery, comprising a tubular body (14) with a relatively inelastic part (20), said inelastic part (20) having an inner layer (30) defining an inner face (34) of the inelastic part (20), said inner layer (30) including a plurality of protruding elements (40) spaced apart from one another,
**characterized in that** the tubular body (14) also comprises a relatively elastic part (22) extending from a first axial end (27) to an opposite axial end (28) of the tubular body (14) and connecting a first edge (60) of the inelastic part (20) to a second edge (62) of the inelastic part (20), and **in that** the item of clothing (10) further comprises:
- a relatively inelastic flap (70) having an inner layer (74) defining an inner face of the flap (70), said inner layer (74) including a plurality of protruding elements (40) spaced apart from one another, the flap (70) being secured to at least part of the first edge (60) of the inelastic part (20), and
- a device (72) for reversibly attaching the flap (70) to at least part of the second edge (62) of the inelastic part (20), designed such that the flap (70) overlaps the elastic part (22) when it is attached.

2. An item of clothing (100) for the treatment of skin and subcutaneous tissue disorders and sports recovery, comprising a tubular body (14) with a plurality of relatively inelastic parts (20) spaced apart from one another and each having an inner layer (30) defining an inner face (34) of said inelastic part (20), said inner layer (30) including a plurality of protruding elements (40) spaced apart from one another,
**characterized in that** the tubular body (14) also comprises a plurality of relatively elastic parts (22), each extending from a first axial end (27) to an opposite axial end (28) of the tubular body (14) and each connecting a first edge (60) of an inelastic part (20) to a second edge (62) of another inelastic part (20), and **in that** the item of clothing (10) further comprises, for each elastic part (22):
- a relatively inelastic flap (70) having an inner layer (74) defining an inner face of the flap (70), said inner layer (74) including a plurality of protruding elements (40) spaced apart from one another, the flap (70) being secured to at least part of the first edge (60) of the inelastic part (60), and
- a device (72) for reversibly attaching the flap (70) to at least part of the second edge of the inelastic part (62), designed such that the flap (70) overlaps the elastic part (22) when it is attached.

3. The item of clothing (100) according to claim 2, wherein the elastic and inelastic parts (20, 22) are arranged alternating with one another.

4. The item of clothing (10, 90, 100) according to any one of the preceding claims, wherein the or each inelastic part (20) and the or each flap (70) each comprise an outer layer (32, 76) made from a relatively inelastic material to which the inner layer (30, 74) is fastened.

5. The item of clothing (10, 90, 100) according to claim 4, wherein the inner layer (30, 74) is fastened to the outer layer (32, 76) by gluing.

6. The item of clothing (10, 90, 100) according to claim 4 or 5, wherein the outer layer (32, 76) is made from a woven or knitted material.

7. The item of clothing (10, 90, 100) according to any one of the preceding claims, wherein the reversible attaching device (72) comprises at least one primary fastening member (80) secured to the flap (70) and at least one secondary fastening member (82) secured to the second edge of the inelastic part (62), the or each primary fastening member (80) being suitable for cooperating with a respective secondary fastening member (82) so as to allow attachment of the flap (70) to the second edge of the inelastic part (62) in different positions.

8. The item of clothing (10, 90, 100) according to claim 7, comprising a device (78) for identifying the position of the flap (70) relative to the second edge of the inelastic part (62).

9. The item of clothing (10, 90, 100) according to claim 8, wherein the identification device (78) comprises a first identifying mark (86) borne by the flap (70), by the inelastic part, respectively, and a plurality of second identifying marks (88) borne by the inelastic part (20), by the flap, respectively, each second identifying mark (88) being associated with a unique attaching position of the flap (70) to the second edge (62), the first identifying mark (86) being designed so as, in each attaching position of the flap (70) to the second edge (62), to be arranged matching the second reference mark (88) associated with said position.

10. The item of clothing (10, 90, 100) according to any one of the preceding claims, wherein the attaching device (72) is a hook-and-loop fastener.

11. The item of clothing (10, 90, 100) according to any one of the preceding claims, wherein the protruding elements (40) comprise pieces of foam, three-dimensional knitted squares, pads filled with liquid, or solid polymer slabs.
